# DEMANDE DE BREVET EUROPEEN

(11) **EP 2 174 673 A1**
(43) Date de publication de la demande: **14.04.2010**
(21) Numéro de dépôt: 09172279.3
(22) Date de dépôt: 06.10.2009
(51) Int. Cl.: A61L 11/00

(54) **Appareil de pré-traitement des dechets à risques infectieux**

(30) Priorité: 09.10.2008 FR 0805563
(71) Demandeur: Ingenierie Etudes et Developpements Industriels (IEDI), 66000 Perpignan (FR)
(72) Inventeur: Raturat, Eric, 66000, PERPIGNAN (FR)
(74) Mandataire: Ravina, Bernard

(57) **Abrégé**

L'invention a pour objet un appareil de pré-traitement des déchets à risques par désinfection comprenant :
- une enceinte de traitement pouvant accueillir un bac apte à contenir les déchets, dotée d'une porte étanche,
- des moyens de chauffage des déchets par rayonnement électromagnétique, et
- des moyens d'alimentation en eau de l'enceinte,
lequel appareil comprend des moyens d'injection d'une quantité d'eau sur les déchets présents dans ledit bac et des moyens de compression desdits déchets, lesdits moyens de compression comportant un plateau mobile (5) dans lequel sont ménagés lesdits moyens d'injection.

Est également revendiqué un procédé de désinfection à l'aide dudit appareil, dans lequel on se place aux conditions de palier adéquates pour réaliser la désinfection, c'est-à-dire pour atteindre l'abattement des germes au niveau souhaité. Durant le palier il est recommandé de rester à une pression sensiblement constante, qui induit un phénomène d'alternance vapeur sèche/vapeur saturante, et pour laquelle le couple pression - température permet d'aboutir à un niveau d'abattement des germes répondant aux normes.

## Description

La présente invention se rapporte au domaine du traitement des déchets d'activités médicales et paramédicales à risques infectieux.

Elle a pour objet un appareil de pré-traitement des déchets à risques par désinfection en conditions contrôlées jusqu'à un niveau d'abattement des germes répondant aux normes en vigueur. Un procédé original pouvant être mis en oeuvre sur le lieu de production des déchets, grâce à cet appareil, est également revendiqué.

Sont considérés comme déchets d'activités de soins à risques infectieux (DASRI) et donc dangereux, ceux qui contiennent des micro-organismes pouvant causer la maladie chez l'homme ou chez d'autres être vivants. Répondent à cette définition, les matériaux piquants ou coupants destinés à l'abandon, les produits sanguins abandonnés, les déchets anatomiques humains non aisément identifiables. En pratique, on trouve dans les matériaux évacués des établissements médicaux un grand nombre d'éléments solides comme pansements, bandages, aiguilles et seringues, couches, cathéters, gants, etc. ..., avec des fractions liquides variables (eau, urines, sérum, alcool, éther, ...).

Par le décret du 18 novembre 1996 sur l'élaboration de plans d'élimination des déchets industriels spéciaux, le législateur français a responsabilisé les producteurs de déchets, en imposant le tri à la source et l'obligation soit d'incinération soit de dépôt en centre d'enfouissement technique de classe 2, assortie de règles d'entreposage précises avec utilisation d'emballages à usage unique et normalisés. Dans ce cadre, la technique de désinfection est admise en tant que traitement préalable à l'incinération.

A côté des méthodes reposant sur la désinfection chimique, par chaleur sèche ou par rayonnements ionisants, qui sont mal adaptées au traitement des DASRI soit pour des raisons de coûts, soit pour des raisons d'efficacité sur des déchets volumineux, on trouve les techniques de désinfection à chaud et sous pression. Selon cette technique, la désinfection est réalisée en chauffant les déchets dans une enceinte close à une température adéquate pour que la vapeur générée entraîne une augmentation de la pression, ces conditions étant suffisantes pour atteindre après un temps donné un niveau d'abattement bactérien et viral conforme aux normes en vigueurs. La conjonction des trois paramètres primordiaux (temps, température, pression de vapeur) couramment admise est en moyenne de l'ordre de 15 minutes à 140°C et 4 bars (400 kPa).

La vapeur peut être produite par un générateur externe ou par chauffage d'un volume d'eau introduit dans la cuve de désinfection, ce qui nécessite son branchement sur le réseau d'eau du local. En outre, l'eau résiduelle est habituellement éliminée en fin de processus par évacuation dans les eaux usées. Selon une autre technique, c'est l'eau contenue dans les déchets eux-mêmes qui permet de produire la vapeur lorsqu'ils sont chauffés. Dans ce cas, la teneur en eau des déchets est variable et difficilement évaluable pour chaque lot, d'où il apparaît soit une difficulté à générer de la vapeur si les déchets sont trop secs, soit une difficulté à décontaminer les germes présent dans l'eau restée en phase liquide.

La montée en température peut se faire par contact, par exemple à l'aide d'une plaque dotée de résistances électriques avec laquelle on compresse les déchets, ou par rayonnement électromagnétique (micro-ondes), le chauffage par contact ayant une efficacité moindre. Une réduction du volume final des déchets est le plus souvent obtenue par compression, cette action mécanique étant généralement couplée à un moyen de chauffage par contact, ceci afin d'optimiser le temps de traitement.

On connaît par exemple la publication US 5124125 qui décrit un procédé de traitement de déchets infectieux, dans lequel les déchets sont placés dans la cuve sous pression d'un dispositif à micro-ondes. Une quantité d'eau est injectée dans la cuve et transformée en vapeur au moyen de micro-ondes, afin d'élever la température à l'intérieure de la cuve au moins à 150°C et la pression au moins à 54 PSIG (soit 372 kPa). Les déchets désinfectés sont compactés par fusion de façon à former un bloc avec la matière plastique qu'ils contiennent, le bloc étant ensuite acheminé vers un broyeur.

On connaît aussi selon FR 2 744 636 et FR 2 767 700, un procédé de désinfection thermique réalisé en compactant le déchet dans une presse chauffante et étanche aux gaz. Le piston de compactage ferme la chambre de manière étanche, la pression pouvant atteindre des valeurs de l'ordre de 10 bars (1000 kPa) pour un traitement à 180°C. Pour améliorer le chauffage par contact, les fonctions de chauffage et de compression sont liées. Selon une variante, aucun apport de liquide n'est fait, considérant que les déchets de type DASRI contiennent assez d'eau ou de liquides assimilés pour générer suffisamment de vapeur et provoquer l'élévation de pression requise. Cependant, cette affirmation est loin d'être vérifiée, les DASRI ayant des compositions fort variables d'un producteur à l'autre, et même d'un lot à l'autre.

Dans tous les cas, le traitement des DASRI y compris leur prétraitement, est réalisé par des sociétés prestataires, qui transportent les déchets depuis le lieu de production et utilisent des équipements de grand volume, pilotés par des personnels spécialement formés. Ces solutions sont loin d'être optimales, tant en termes énergétiques, qu'en termes de sécurité et de coûts. Elles sont de manière générale inadaptées pour des producteurs de petites et moyennes quantités de déchets. Leur coût est imputable à plusieurs postes : les dépenses énergétiques pour le chauffage, les coûts des récipients de conditionnement et surtout le transport du site de production à celui de traitement ainsi que les dépenses de personnel.

Il est donc apparu indispensable de proposer un mode de traitement des déchets d'activité de soins à risques infectieux répondant aux exigences d'une meilleure gestion des déchets du monde médical dans sa diversité, du respect de l'environnement, de l'hygiène et de la santé, tout en offrant des conditions économiquement incitatives.

C'est pourquoi la présente invention définit une nouvelle façon de traiter ce type de déchets : décontaminer au plus tôt et au plus prêt de la source les déchets à risques, par une machine de taille adaptée aux besoins journaliers des utilisateurs, facile à installer, simple d'emploi et de maintenance, et ne nécessitant pas d'infrastructure de fonctionnement particulière (eau, air comprimé, évacuation etc..), selon un procédé de fonctionnement conforme à la norme NF X30-503. En particulier, le nouvel équipement proposé a les caractéristiques suivantes :
- la taille de l'appareil est suffisamment faible pour qu'il puisse être placé dans tout type de local, même réduit s'il est ventilé. Il peut avoir les dimensions d'un réfrigérateur grand public.
- son installation est immédiate : seul un raccordement électrique est nécessaire au fonctionnement de l'appareil, il n'a pas besoin d'arrivée et d'évacuation d'eau.
- son utilisation est suffisamment simple pour qu'un opérateur sans formation spécifique soit capable de l'utiliser.
- il permet d'utiliser un récipient de conditionnement conforme aux normes NF X30-505 et NF X30-506.
- la durée d'un cycle de désinfection est court, inférieur à deux heures.
- l'entretien et la maintenance sont réduits, le nettoyage intérieur est facile et rapide.
- le procédé est économe en énergie.

Ce dernier objectif est réalisé par une optimisation des conditions de température et de pression tout au long du processus de désinfection. Cet aspect sera illustré en détail dans la description qui va suivre. A noter que les termes traitement et prétraitement seront souvent utilisés par la suite comme des synonymes, étant bien entendu que la présente invention se rapporte à un appareil de désinfection des déchets à risques (et au procédé correspondant) qui constitue une étape préalable à leur incinération au même titre que les déchets d'ordures ménagères.

Plus précisément, est objet de l'invention un appareil de pré-traitement des déchets à risques par désinfection comprenant :
- une enceinte de traitement pouvant accueillir un bac apte à contenir les déchets, dotée d'une porte étanche,
- des moyens de chauffage des déchets par rayonnement électromagnétique, et
- des moyens d'alimentation en eau de l'enceinte, lequel appareil comprend également des moyens d'injection d'une quantité d'eau sur les déchets présents dans le bac et des moyens de compression desdits déchets, lesdits moyens de compression comportant un plateau mobile dans lequel sont ménagés lesdits moyens d'injection.

L'enceinte, de type connu, délimite un espace qui constitue une chambre de traitement, dont le volume définit la capacité de traitement de l'appareil. L'utilisation de matériaux inoxydables et résistants aux produits couramment utilisés dans le domaine médical est indispensable. Une isolation thermique est également à prévoir. La porte étanche est de taille suffisante pour introduire un bac dans l'enceinte. Le bac est choisi parmi les récipients rigides connus pour cet usage, et tels que définis par les normes se rapportant au conditionnement des déchets DASRI. Dans ce qui suit, l'enceinte désignera généralement aussi bien la structure délimitant la chambre de traitement que la chambre elle-même.

Les moyens de chauffage par rayonnement électromagnétique sont communément réalisés par un magnétron émettant des micro-ondes. De manière avantageuse, le magnétron peut être doté d'un guide d'ondes à déflecteur variable et d'une plaque de transfert hautes fréquences.

Les moyens de compression des déchets comprennent typiquement une presse, par exemple de type servo-presse intégrant par construction des capteurs de force et de déplacement, dont le corps est extérieur à l'enceinte. La presse actionne le plateau de compression par un axe extensible (par exemple par coulissement traversant la paroi de l'enceinte par une liaison étanche. Le plateau lui-même n'est pas en contact avec les parois de la cuve, et contrairement à un piston, il ne constitue pas un obstacle à la circulation de l'air à l'intérieur de la chambre de traitement. De ce fait, lorsque la presse est actionnée, le plateau descend dans la chambre de traitement sans créer de différence de pression de part et d'autre. En effet, selon l'invention, la compression est principalement destinée à réduire le volume des déchets par tassement après leur traitement, elle ne contribue pas à proprement parler au traitement thermique des déchets en provoquant une montée en pression de la chambre ou un transfert calorique par contact avec les déchets.

L'eau nécessaire au traitement est déversée sur les déchets présents dans le bac par des moyens d'injection, qui sont reliés à des moyens d'alimentation en eau de l'enceinte. Ces moyens d'injection sont ménagés dans le plateau mobile, de sorte que celui-ci remplit deux fonctions indépendantes tout en occupant un espace réduit. Les étapes d'injection d'eau dans l'enceinte et de compression des déchets sont réalisées selon le procédé inventif généralement de manière successive et distincte.

La configuration combinée des moyens d'injection d'eau et de compression offre un système dont un avantage est sa compacité, mais qui surtout est compatible avec d'autres fonctions particulièrement intéressantes mises en oeuvre dans l'appareil objet de l'invention, ou même qui est une condition permettant leur réalisation.

Dans l'appareil selon l'invention, les moyens d'injection d'une quantité d'eau ménagés dans le plateau mobile comprennent avantageusement un conduit et au moins une buse débouchant à la surface inférieure dudit plateau mobile, ledit conduit étant relié par un tuyau extensible au circuit d'amenée d'eau des moyens d'alimentation en eau placés à l'extérieur de l'enceinte. Bien que le plateau mobile puisse comporter une seule buse, il est bien sûr préférable qu'il en comporte au moins deux, et de préférence au moins quatre. Le tuyau extensible est réalisé en un matériau résistant et souple de type silicone, et peut s'étirer pour suivre les mouvements du plateau mobile dans toute son amplitude.

Les moyens d'alimentation en eau sont constitués essentiellement d'un réservoir alimentant l'enceinte par l'intermédiaire du circuit d'amenée d'eau susmentionné, la circulation d'eau pouvant commodément être contrôlée par une pompe d'injection et une électrovanne. Le réservoir peut être réfrigéré, de sorte que sa température reste inférieure la température ambiante, et soit de préférence inférieure à 10°C. Il peut en outre être opaque.

Ainsi, selon une caractéristique intéressante de l'appareil selon l'invention, les moyens d'alimentation en eau de l'enceinte comprennent un réservoir réfrigéré et un circuit d'amenée d'eau muni d'une pompe d'injection et d'une électrovanne.

Selon une autre caractéristique intéressante, l'appareil objet de l'invention comprend un circuit d'apport d'air froid dans l'enceinte, muni d'une électrovanne, de préférence de type ouvert - fermé. De manière avantageuse, le circuit d'apport d'air froid dans l'enceinte prend son origine dans la partie supérieure du réservoir réfrigéré, au-dessus du niveau de l'eau. L'eau et l'air sont ainsi réfrigérés par un même et unique dispositif. Selon un mode de réalisation particulier, le réservoir peut être doté d'un module à effet Peltier, d'un capteur de température et de détecteurs des niveaux minimum et maximum autorisés de l'eau.

On dispose ainsi d'une réserve de fluides (eau et air) frais, qui peuvent notamment être introduits dans l'enceinte avec le bénéfice qui sera présenté plus loin. Une telle disposition présente en outre l'avantage de prévenir le développement des microorganismes dans le réservoir et par suite la contamination dans toute l'installation.

Dans un mode de réalisation particulièrement avantageux de l'invention, l'appareil comprend des moyens de contrôle et de régulation de la pression interne de l'enceinte, lesdits moyens comprenant :
- au moins un capteur de la pression interne,
- un circuit de mise en dépression de l'enceinte muni d'une pompe à vide et d'une électrovanne, par exemple de type ouvert - fermé,
- un circuit d'extraction de la vapeur formée dans l'enceinte, doté de préférence d'une électrovanne progressive, et
- un circuit de sécurité muni d'une soupape de surpression.

L'ensemble de ces éléments permet de faire varier la pression dans l'enceinte, avant, pendant et après le déroulement de la désinfection, afin d'optimiser les conditions thermodynamiques de traitement, ce qui concourt à la qualité du traitement pour une dépense énergétique minimum, et à la sécurité de fonctionnement de l'appareil. Certaines phases peuvent ainsi être effectuées sous pression réduite, ce qui permet d'abaisser la température de vaporisation des liquides. Lorsque l'eau passe à l'état de vapeur, la pression peut être maintenue à la pression de vapeur saturante minimum, de sorte que toute l'eau soit transformée en vapeur. La pression de vapeur saturante minimum s'entend ici comme étant une limite basse de la pression de vapeur saturante. Puis, durant le palier il est recommandé selon l'invention de rester à une pression sensiblement constante, qui induit un phénomène d'alternance vapeur sèche/vapeur saturante, et pour laquelle le couple pression - température permet d'aboutir à une désinfection efficace.

Selon une caractéristique préférée de l'invention, le circuit d'extraction de la vapeur pénètre dans le réservoir de sorte que son orifice de sortie débouche sous le niveau de l'eau. On comprend aisément que dans la mesure où le réservoir est maintenu à une température basse, la vapeur s'échappant de l'enceinte par ce circuit va se condenser en arrivant dans le réservoir et s'y écouler, contribuant ainsi à reconstituer la réserve d'eau destinée à être injectée sur les déchets lors du cycle suivant. On comprend également l'intérêt de disposer sur le circuit d'extraction de la vapeur, d'une électrovanne progressive dont l'ouverture sera fonction de la pression que l'on souhaite maintenir dans l'enceinte.

Selon un mode de réalisation préféré de l'invention, les moyens de compression comprennent une presse électrique dont le plateau mobile a une surface inférieure à l'ouverture du bac. Comme déjà mentionné, la presse a pour fonction première de tasser les déchets en fin de traitement, afin d'en réduire le volume. Selon l'invention, il est proposé de tasser les déchets à l'intérieur du bac, sans écraser celui-ci comme c'est le cas dans la plupart des techniques connues, grâce à un plateau de presse capable d'y pénétrer et de compresser les déchets dans le fond du bac. Il est alors possible d'utiliser un même bac pour plusieurs cycles, en le rechargeant de nouveaux déchets entre chaque cycle, jusqu'à un taux de remplissage satisfaisant. Cette disposition présente un avantage économique certain, quand on connaît le prix des récipients de conditionnement.

En outre, dans l'appareil selon la présente invention, le plateau mobile peut comprendre des buses latérales aptes à projeter de l'eau sur les parois latérales de l'enceinte. Ainsi, entre deux cycles de traitement, il est possible d'effectuer un cycle de nettoyage en injectant de l'eau sous pression dans l'enceinte tout en faisant circuler le plateau mobile de haut en bas et de bas en haut. L'évacuation de l'eau et le séchage pourront être menés selon le même principe que les étapes de vaporisation et de déshydratation du traitement des déchets.

Selon un mode de réalisation particulier, l'appareil objet de l'invention comporte des moyens de détection de la présence d'un bac et de son degré de remplissage, lesdits moyens de détection étant constitués d'une plaque support sur laquelle le bac est destiné à être posé, celle-ci reposant sur des ressorts tarés avec butée, et des moyens de mesure et d'analyse de la force de pression exercée par le plateau mobile de la presse en fonction de son déplacement. La plaque support est semi mobile dans la mesure où elle ne peut se déplacer que sur la course des ressorts tarés avant la butée. Les ressorts sont bien entendu choisis pour jouer leur rôle dans l'intervalle de poids à mesurer. Ces organes, bien que ne participant pas directement au processus de désinfection, permettent un fonctionnement en toute sécurité de l'appareil objet de l'invention, qui a justement été conçu pour une utilisation automatisée sans intervention d'un personnel spécialisé.

Pour les mêmes raisons, il sera prévu en général d'automatiser le paramétrage de l'appareil et de lui adjoindre des systèmes de coupure en cas de dysfonctionnement. Une interface utilisateur conviviale sera appréciée également. Enfin, on pourra inclure dans les fonctions automatiques, des moyens d'acquisition et de mémorisation des cycles effectués, qui pourront être pris en compte pour moduler les paramètres de fonctionnement des cycles ultérieurs. Ainsi, selon une autre mode de réalisation particulier l'appareil objet de l'invention peut comprendre un ou plusieurs des moyens suivants :
- un capteur de température dans l'enceinte,
- des moyens de contrôle des paramètres de fonctionnement de l'appareil,
- des moyens d'acquisition des paramètres de fonctionnement de l'appareil,
- des moyens informatiques de pilotage avec panneau de contrôle visuel.

L'appareil tel qu'il vient d'être décrit est particulièrement adapté à la mise en oeuvre d'un procédé de traitement des déchets médicaux à risques, ce procédé étant également objet de l'invention. Est ainsi revendiqué un procédé de pré-traitement des déchets infectieux par désinfection, comprenant essentiellement les étapes consistant à :
a) déposer les déchets à stériliser dans un bac placé dans l'enceinte d'un appareil de pré-traitement tel que décrit précédemment, qui comprend des moyens d'injection d'une quantité d'eau sur les déchets et des moyens de compression desdits déchets dotés d'un plateau mobile dans lequel sont ménagés lesdits moyens d'injection ;
b) fermer la porte étanche de l'enceinte ;
c) injecter une quantité d'eau sur les déchets présents dans le bac par les moyens d'injection ménagés dans le plateau mobile ;
d) soumettre les déchets à un rayonnement électromagnétique jusqu'à réalisation des conditions de palier température - pression - temps adéquates pour réaliser la désinfection,
e) abaisser le plateau mobile des moyens de compression dans le bac pour compresser les déchets ainsi traités ;
f) faire refroidir l'enceinte avant de déverrouiller la porte.

Les conditions de palier adéquates pour réaliser la désinfection sont les valeurs des paramètres température, pression et durée à appliquer pour effectuer la désinfection, c'est-à-dire pour atteindre l'abattement des germes au niveau souhaité, ce niveau étant au moins conforme à la législation en vigueur. Comme déjà indiqué plus haut, les normes ne précisent pas la valeur des paramètres, mais seulement l'abattement minimum à atteindre, qui doit être en France d'au moins 10⁴, tous microorganismes confondus. Dans la pratique, ces valeurs sont définies sans difficulté par l'homme de l'art à partir de ses connaissances professionnelles. Dans le cas d'espèce, elles sont avantageusement programmées dans une unité de pilotage de l'appareil.

Dans un mode particulier de mise en oeuvre, le procédé objet de la présente invention peut comprendre, avant l'étape c) d'injection de l'eau, une étape c₂) d'élimination des composés dont la tension de vapeur est supérieure à celle de l'eau, au cours de laquelle on réduit la pression dans l'enceinte, on soumet les déchets à un rayonnement électromagnétique jusqu'à vaporisation desdits composés, et on purge les vapeurs formées par une nouvelle réduction de la pression dans l'enceinte. Le but est d'éliminer tous les composés se vaporisant à une température inférieure à celle de l'eau, pour éviter que n'apparaissent lors de la désinfection, des pressions de vapeurs partielles qui ne seraient pas celles de la vapeur d'eau. On applique donc pour cette étape une température de chauffage provoquant la vaporisation des éthers et des composés éthyliques à la pression imposée. Par exemple pour une pression imposée de 150 mbar (15 kPa), les éthers et composés éthyliques s'évaporent vers 35°C au plus.

Selon une caractéristique préférée, l'étape c₂) comprend une étape préalable c₁) de rafraîchissement de l'enceinte, au cours de laquelle on purge d'abord l'air de l'enceinte à l'aide d'un circuit de mise en dépression muni d'une pompe à vide, puis on apporte de l'air froid par un circuit d'apport d'air froid. Le rafraîchissement est une étape qui a pour but d'abaisser le plus possible la température de l'atmosphère de l'enceinte. On estime que plus l'oxygène est rare (dépression) et plus la température est basse, moins il y aura de risque d'inflammation, surtout si certains déchets sont en inox, ce qui provoque au tout départ de la mise en route du magnétron, le phénomène d'étincelle que l'on constate habituellement dans un système à micro-ondes. Il n'est ainsi plus nécessaire de trier les déchets métalliques avant leur désinfection, ce qui évite des manipulations laborieuses et risquées, et représente un gain de temps très appréciable.

Dans un mode particulier de mise en oeuvre du procédé selon l'invention, à l'étape c), on injecte une quantité d'eau fixe, prédéterminée en fonction des dimensions de l'appareil. Il a en effet été constaté que si certains déchets contiennent de l'eau, sa quantité n'est souvent pas suffisante. Or, il est difficile de modéliser le comportement d'un mélange de déchets de compositions et de natures différentes (coton, matières plastiques, métal, matières organiques, ... pour les solides ; éther alcool, liquides organiques, eau, ... pour les liquides). Pour contourner ce problème, il a été calculé le volume d'eau minimum permettant de générer la quantité de vapeur nécessaire au traitement d'un mélange de déchets totalement dépourvus d'eau. Il est ainsi apparu que même si, ce faisant, on ajoute de l'eau en excès sur des déchets en contenant déjà, l'énergie supplémentaire nécessaire au cours du processus (pour chauffer, évaporer, déshydrater) était peu significative au regard du gain obtenu par le fonctionnement en dépression et par la simplification et la standardisation du procédé. Ainsi, pour un appareil donné dont l'enceinte est apte à recevoir une quantité de déchets donnée par cycle de traitement, le volume d'eau introduit dans l'enceinte sera programmé par défaut à une valeur fixe préétablie. Il a été déterminé que, par exemple pour une enceinte de 80 litres, pouvant recevoir un bac de 30 litres de déchets par cycle, le volume d'eau optimal était de l'ordre de 200 cm³, soit 0,25 % du volume de l'enceinte. De même, dans une enceinte de 180 litres, 440 cm³ d'eau seront ajoutés sur les déchets contenus dans un bac dont la contenance prévue est de 60 litres.

Selon une caractéristique intéressante du procédé objet de la présente invention, durant l'étape d) de désinfection, la pression de vapeur dans l'enceinte est maintenue à une valeur sensiblement constante en évacuant une partie de la vapeur formée. A noter que ceci est rendu possible grâce au fait que le plateau de la presse n'est pas en contact avec les parois de la chambre de traitement, et qu'ils ne gênent pas l'équilibre des pressions dans tout l'espace interne de la chambre. Cette étape de désinfection effective des déchets est appelée palier de désinfection. Il est couramment admis qu'un traitement de 20 mn des déchets à une température de l'ordre de 145°C et à une pression de l'ordre de 4,4 bars (440 kPa), correspond aux conditions moyennes pour lesquelles une désinfection efficace est constatée. A côté des valeurs propres retenues pour chaque paramètre, la conjonction constante du triplet température - pression de vapeur - temps est essentielle. En pratique, la pression oscillera autour d'une valeur moyenne, entre le seuil de pression de vapeur saturante et le seuil de pression de vapeur sèche à la température régnant dans la chambre de traitement.

Selon une caractéristique particulièrement intéressante du procédé objet de la présente invention, au début de l'étape d) de désinfection, la pression est maintenue dans l'enceinte à la pression de vapeur saturante minimum, de sorte que toute l'eau soit transformée en vapeur. Dans cette phase dite d'avant-palier, la pression est régulée de manière à rester en permanence en limite entre la pression de vapeur sèche PVsec (où toute l'eau est transformée en vapeur) et la pression de vapeur saturante PVsat (où l'eau est sous forme mixte vapeur et liquide). Ceci peut être réalisé par exemple, par la régulation de l'ouverture de l'électrovanne du circuit d'extraction de la vapeur (faible fuite). Cette étape dont la contribution innovante est importante, poursuit deux buts :
- éviter que des virus ou bactéries puissent être présents dans l'eau à l'état liquide et donc échapper à la désinfection qui suivra ;
- réduire la quantité de liquide dans l'enceinte pour anticiper l'étape ultérieure de déshydratation des déchets.

Selon une caractéristique avantageuse du procédé selon l'invention, l'eau injectée dans l'enceinte à l'étape c) est stockée dans un réservoir réfrigéré à une température inférieure à la température ambiante, de préférence inférieure à 10°C. Outre les avantages déjà décrits plus haut, là aussi, la prolifération des germes est freinée par le froid.

Selon une autre caractéristique avantageuse, à l'étape c₁) de rafraîchissement de l'enceinte, l'air froid est prélevé dans la partie supérieure du réservoir réfrigéré, au-dessus du niveau de l'eau, par l'effet de la différence de pression entre le réservoir et l'enceinte. Cette disposition est rendue possible et commode du fait de la température réduite du réservoir.

De manière avantageuse, selon l'invention, durant l'étape d) de désinfection, la vapeur d'eau produite dans l'enceinte est évacuée par un circuit d'extraction de la vapeur plongeant dans le réservoir réfrigéré de sorte qu'elle se condense et s'y déverse. L'eau mise en oeuvre aux différentes étapes est ainsi recyclée dans le circuit. Il n'est plus nécessaire de relier l'appareil à une arrivée d'eau, il suffit de le remplir au niveau requis lors de sa première mise en service.

Selon une caractéristique intéressante, le procédé objet de la présente invention comprend, immédiatement après l'étape d) de désinfection, une étape d₁) de déshydratation des déchets sous pression réduite. Cette étape se déroule alors que la température de l'enceinte est encore élevée, avec mise en dépression de l'enceinte, ce qui contribue à éliminer la plus grande partie de l'humidité. La mise en dépression de l'enceinte peut être réalisée à l'aide de la pompe à vide du circuit de mise en dépression.

L'étape d'assèchement des déchets est une fonction innovante du procédé qui participe à la sécurité et à la qualité du traitement. En effet, en éliminant l'environnement aqueux, elle évite une revivification bactérienne et virale rapide. Elle a aussi pour vocation d'augmenter le pouvoir calorifique inférieur (PCI) des déchets pour leur traitement comme ordures ménagères dans une usine d'incinération. Un poids final des déchets légèrement abaissé, optimise également le taux de remplissage du conteneur et minimise les coûts de traitement.

Selon une autre caractéristique intéressante du procédé objet de l'invention, l'étape e) de compression est réalisée par descente du plateau mobile de la presse à l'intérieur du bac. De préférence, on opère alors que l'enceinte est restée en dépression. Ainsi, en fin de cycle, seuls les déchets sont écrasés et tassés dans le bac, le bac étant lui-même préservé. Il peut être rechargé pour subir un nouveau cycle de désinfection.

Selon un mode de réalisation particulier de l'invention, durant l'étape f) de refroidissement de l'enceinte, on purge d'abord l'air de l'enceinte en actionnant la pompe à vide du circuit de mise en dépression, puis on apporte de l'air froid par le circuit d'apport d'air froid, ces opérations étant répétées jusqu'à atteindre la température finale souhaitée. Ceci accélère le refroidissement des déchets et permet d'enchaîner rapidement plusieurs cycles dans la journée. La température finale recherchée est celle qui permet à l'utilisateur de manipuler le bac sans se brûler au contact du bac ou des parois de l'enceinte.

De manière intéressante, le procédé selon l'invention peut comprendre une étape de détection de la présence d'un bac et/ou de son degré de remplissage, à l'aide d'une plaque support sur laquelle le bac est supposé être posé, celle-ci reposant sur des ressorts tarés avec butée, et des moyens de mesure et d'analyse de la force de pression exercée par le plateau de la presse mobile lors de son déplacement.

Le test de présence d'un conteneur est effectué en analysant la courbe de force appliquée par la servo-presse en fonction de son déplacement. Il évite de faire fonctionner l'appareil à vide. Par ailleurs, si le volume détecté ne justifie pas de lancer le traitement, il est possible de stocker temporairement les déchets, pour mettre en route l'appareil lorsque le bac sera suffisamment plein. On pourra prévoir durant ce stockage, un rafraîchissement périodique de l'atmosphère de l'enceinte et un maintien en dépression (réduction de l'oxygénation) minimisant ainsi la prolifération microbienne.

Egalement, selon un mode de réalisation particulier, le procédé objet de l'invention peut comprendre entre deux cycles de traitement, une étape de nettoyage des parois latérales de l'enceinte par descente du plateau mobile et projection d'eau par des buses latérales.

Un tel équipement peut être installé avec les mêmes avantages, aussi bien dans le secteur hospitalier, produisant une quantité de déchets supérieure à 100 kg par semaine, que dans des structures plus petites telles que les laboratoires d'analyse, les centres de santé, les maisons de retraite, les cabinets médicaux et vétérinaires produisant entre 5 kg par mois et 100 kg par semaine.

Le procédé selon l'invention permet de réduire *in situ* le volume et la dangerosité des déchets transportés avec un niveau de risques considéré comme nul selon les normes et agréments en vigueur concernant le taux d'abattement de la contamination bactérienne et virale. Le déchet ainsi pré-traité est assimilable à un déchet d'ordure ménagère, et peut suivre les voies de traitement plus classiques avec des coûts d'exploitation correspondants. Le prix de revient de traitement brut est inférieur à 350 € par tonne en première estimation. Les coûts de transport sont nuls puisque les déchets désinfectés sont assimilables aux ordures ménagères, dont les coûts de collecte sont déjà assumés par l'établissement. A noter que le Conseil National d'Hygiène Publique de France a reconnu la technique de prétraitement par désinfection comme une alternative acceptable. Elle admet également la possibilité d'une désinfection *in situ,* directement sur le lieu de production.

D'autres avantages et caractéristiques de l'invention apparaîtront à la lecture de la description d'un mode de réalisation, donné à titre d'exemple non limitatif, illustré par les figures 1 et 2 qui sont des vues schématiques d'un appareil selon l'invention, respectivement de face et de côté. La figure 3 illustre l'évolution des conditions de température et de pression au cours d'un cycle de traitement.

### EXEMPLE 1 - APPAREIL DE TRAITEMENT 30 LITRES

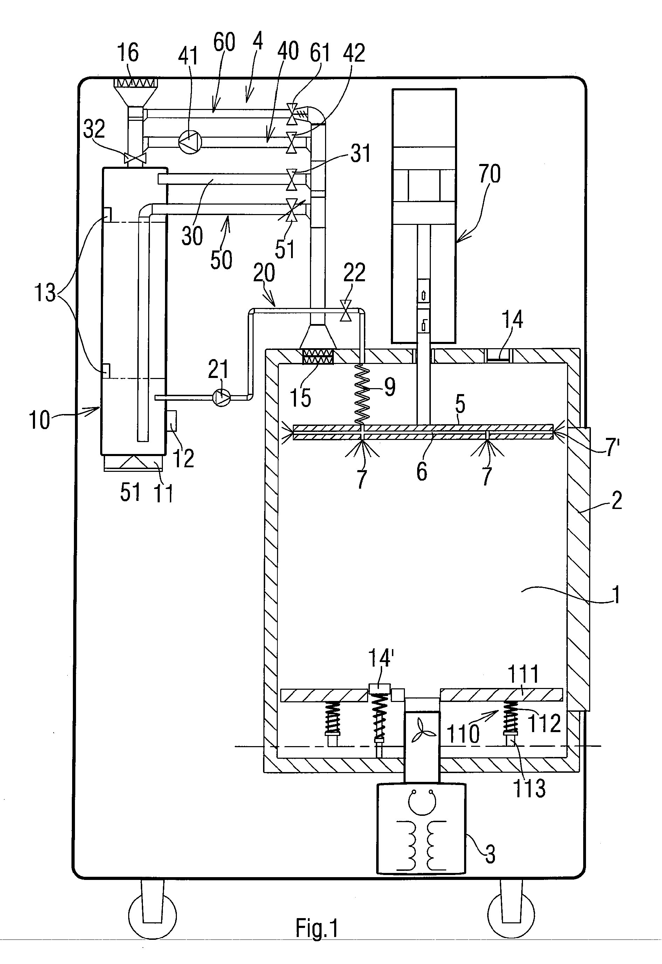

L'appareil de pré-traitement des déchets à risques par désinfection représenté à la figure 1 comprend l'enceinte 1 de traitement pouvant accueillir un bac 100 apte à contenir les déchets; Il est doté de la porte étanche 2, des moyens de chauffage 3 des déchets par rayonnement électromagnétique, et des moyens d'alimentation en eau 4 de l'enceinte. L'enceinte délimite une chambre de traitement d'un volume de 80 litres (ou 180 litres selon un autre modèle), destinée à recevoir un bac de 30 litres de déchets (ou 60 litres). Les moyens de chauffage 3 sont constitués d'un magnétron émettant des micro-ondes, doté d'un guide d'ondes à déflecteur variable et d'une plaque de transfert hautes fréquences.

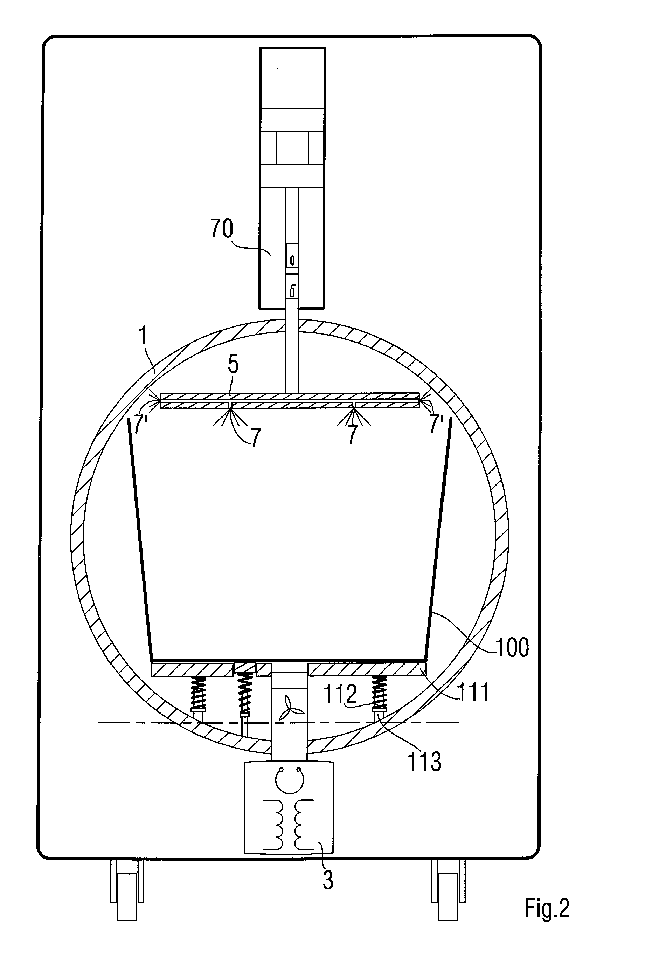

Les moyens de compression des déchets comprennent la presse 70, de type servo-presse, intégrant par construction des capteurs de force et de déplacement, dont le corps est extérieur à l'enceinte 1. La presse 70 actionne le plateau de compression 5 par un axe extensible par coulissement, ici vertical, traversant la paroi de l'enceinte 1 par une liaison étanche. Comme on le voit sur la figure 2, le plateau mobile 5 a une dimension telle qu'il peut être déplacé librement dans l'enceinte 1 selon son axe sans contact avec les parois de l'enceinte 1, ni avec le bac 100. Le bac peut être par exemple un fût rigide standard, tel que ceux commercialisés sous le nom Biogrip^{™} ou Pacazur^{™}.

L'eau nécessaire au traitement est déversée sur les déchets présents dans le bac 100 par les moyens d'injection qui sont ménagés dans le plateau mobile 5. Les moyens d'injections sont formés du conduit 6 et de plusieurs buses 7 débouchant à la surface inférieure du plateau mobile 5. Le conduit 6 est relié par le tuyau extensible 9 au circuit d'amenée d'eau 20 alimenté par le réservoir 10 qui est placé à l'extérieur de l'enceinte. La circulation de l'eau est contrôlée par la pompe d'injection 21 et l'électrovanne 22. Le réservoir 10 est réfrigéré de sorte que sa température reste aux environ de 5°C à l'aide du module à effet Peltier 11. Il est équipé du capteur de température 12 et des détecteurs 13 des niveaux minimum et maximum autorisés de l'eau.

L'appareil objet de cet exemple comprend le circuit 30 permettant d'apporter de l'air froid dans l'enceinte 1. Le circuit d'air froid 30 prend son origine dans la partie supérieure du réservoir réfrigéré 10, au-dessus du niveau de l'eau. Il est muni de l'électrovanne 31, de type ouvert - fermé.

L'appareil ici présenté comprend aussi différents organes destinés à assurer son bon fonctionnement, en autonomie et en toute sécurité.

Le capteur de pression interne 14 détecte les variations barométriques dans la chambre de traitement de l'enceinte. Ce capteur comme le capteur de température 14' sont reliés à un système de pilotage qui peut stopper le traitement en cas d'anomalie.

Le plateau mobile 5 comprend les buses latérales 7' aptes à projeter de l'eau sur les parois de l'enceinte 1 en l'absence de bac.

Le circuit de mise en dépression 40 de l'enceinte 1 est muni de la pompe à vide 41 et de l'électrovanne 42 de type ouvert - fermé. Il assure la purge de l'atmosphère de l'enceinte, et son maintien sous pression réduite.

Le circuit d'extraction 50 de la vapeur formée dans l'enceinte 1 est doté de l'électrovanne progressive 51 dont l'ouverture plus ou moins importante est pilotée de manière à créer une faible fuite afin de réguler la pression dans l'enceinte 1 à un niveau constant durant le traitement. Le circuit 50 pénètre dans le réservoir 10 de sorte que son orifice de sortie 51 débouche sous le niveau de l'eau. La vapeur condensée au contact de la partie froide du conduit s'écoule ainsi dans le réservoir 10.

Le circuit de sécurité 60, muni d'une soupape de surpression 61, assure le maintien de l'appareil à l'intérieur des valeurs limites de fonctionnement.

Les différents circuits gazeux débouchent dans, ou hors de l'enceinte par un conduit unique muni d'éléments de filtration 15 des gaz, notamment d'un filtre amont de rétention des grosses particules et d'un filtre aval haute efficacité bloquant jusqu'au plus petit virus. Les circuits s'évacuant vers l'atmosphère débouchent dans un conduit de sortie unique, équipé du filtre anti-odeurs 16. Toutes les électrovannes sont des modèles "normalement fermée" ou "NF". En cas de coupure de courant, la chambre en phase de pression ne sera pas brutalement dépressurisée mais conservée en l'état.

Enfin, l'appareil objet de l'invention comporte des moyens de détection 110 de la présence d'un bac 100 et de son degré de remplissage. La plaque support 111 sur laquelle le bac 100 est destiné à être posé, repose sur les ressorts tarés 112 avec butée 113. Ils sont associés à des moyens de mesure et d'analyse de la force de pression exercée par le plateau mobile 5 de la presse 70 en fonction de son déplacement.

### EXEMPLE 2 : REALISATION D'UN CYCLE DE PRE TRAITEMENT

Le procédé de traitement des déchets médicaux à risques selon l'invention peut être mis en oeuvre avec l'appareil tel que décrit à l'exemple 1, en réalisant les étapes ci-après détaillées d'un cycle complet, utilisant toutes les options disponibles.

Dans une première étape, on dépose les déchets à désinfecter dans le bac 100 placé dans l'enceinte 1 de l'appareil de pré-traitement tel que décrit précédemment, puis on verrouille la porte étanche 2.

Un test de présence d'un conteneur et/ou de son degré de chargement est effectué, en analysant la courbe de force F appliquée par la servo-presse en fonction de son déplacement D. La presse 70 est activée pour une descente à vitesse lente V1 du plateau de presse 5, jusqu'à atteindre une force F(éval) de pression, puis remontée à vitesse rapide V2 jusqu'à son point haut maximum.

La vitesse V1 est fixée à 4 mm par seconde environ, et la vitesse V2 à 12 mm par seconde. F(éval) est un seuil d'environ 250 N, légèrement supérieur au poids maximum de déchets admissibles. On mesure constamment la force F et le déplacement D. Selon l'analyse de la courbe F = f(D), l'appareil est mis en route ou bien laissé en veille, ce qui permet à l'opérateur de stocker temporairement ses déchets quand le volume ne justifie pas un traitement. Durant cette phase de stockage un rafraîchissement périodique de l'atmosphère de chambre et un maintien en dépression sont assurés, minimisant ainsi la prolifération bactérienne.

L'analyse de la courbe F = f(D) repose sur des abaques prédéterminées à partir de mélanges typiques connus et de caractéristiques intrinsèques à la machine. Un logiciel d'auto apprentissage appliquant les concepts des systèmes experts peut être utilisé pour enrichir les données initiales.

Si l'analyse montre qu'il est nécessaire d'effectuer un cycle de désinfection, l'appareil est mis en route. On procède d'abord à un rafraîchissement de l'enceinte :
1) Extraction de l'air de l'enceinte par le circuit 40 de mise en dépression. La pompe 41 est mise en route et l'électrovanne 42 est ouverture jusqu'à atteindre une pression de la chambre de 300 mbar ± 50 mbar (30 kPa ± 5 kPa).
2) Injection d'air à 5°C par le circuit d'injection d'air 30. Le principe repose sur les différences de pression entre l'enceinte 1 et le réservoir 10.

On procède ensuite à la purge des composés inflammables. L'enceinte est mise en dépression à environ 150 mbar ± 50 mbar (15 kPa ± 5 kPa). Le magnétron est activé pour chauffer le contenu de l'enceinte. A la pression imposée, les éthers et composés éthyliques s'évaporent vers 35°C au plus. Les vapeurs formées génèrent une surpression à partir de la pression initiale imposée (augmentation théorique à 300 mbar (30 kPa) à 50°C). Les gaz sont évacués par le circuit de dépression 40 à l'aide de la pompe 41.

Puis, l'enceinte est replacée en dépression (150 mbar, 15 kPa), et une quantité d'eau fixe de 200 cm³ pour une enceinte de 80 litres est injectée depuis le réservoir 10 par le circuit d'injection de l'eau 20 à l'aide de la pompe 22. Le magnétron 3 est alors mis en route pour chauffer l'enceinte dans une étape dite d'avant-palier, jusqu'à atteindre une température de 147°C et une pression proche du seuil de pression (PVsat eau) s'élevant ici à 4600 mbar ± 50 mbar (460 kPa ± 5 kPa).

On crée alors des conditions de température et de pression pour réaliser la phase de palier, qui est l'étape de désinfection à proprement parler. La température est régulée à 147°C tout en évacuant le surplus de vapeur pour redescendre à une pression avoisinant les 4400 mbar (440 kPa). La conjonction d'une température de 147°C et d'une pression de vapeur de 4400 mbar est un état de vapeur sèche et il n'existe pas d'eau à l'état liquide dans l'enceinte à cet instant. La régulation en pression de vapeur est réalisée en ménageant une ouverture faible de l'électrovanne 51 du circuit 50 d'extraction de vapeur et en évacuant la vapeur par micro fuite vers le réservoir 10 via le circuit 50. On régule la pression pour rester en limite de conditions saturantes.

Le maintien de la température de l'enceinte est réalisé à l'aide du magnétron. Dans cette étape de palier d'une durée de 20 mn, on alterne donc constamment entre les états PVsat et Pvsec, avec une température de l'ordre de 145°C et une pression de l'ordre de 4300 mbar (430 kPa), ce qui correspond à des conditions couramment admises pour une désinfection efficace.

A la fin de la phase de désinfection, on procède à la déshydratation tout en continuant de contrôler le magnétron 3 pour maintenir une température supérieure ou égale à 140°C. On ouvre progressivement de faible à forte l'électrovanne 22 du circuit d'extraction de la vapeur 20 et on évacue la vapeur vers le réservoir 10, jusqu'à ce que l'enceinte soit revenue à la pression atmosphérique.

On procède alors à la déshydratation avec tassement sous vide, alors que la température est encore élevée dans l'enceinte. La presse 70 est activée pour une descente à vitesse lente V1 du plateau mobile 5, jusqu'à atteinte de la force de tassement Ftass prédéterminée (par exemple de l'ordre de 2 500 N), la pression de l'enceinte étant quant à elle amenée à environ 150 mbar ± 50 mbar (15 kPa ± 5 kPa). La presse est maintenue en position durant cette étape.

Finalement, le bac et l'enceinte sont refroidis (ainsi que les déchets désinfectés). De l'air froid est envoyé dans l'enceinte depuis la partie supérieure du réservoir 10 par le circuit 30, la vanne d'entrée d'air 32 étant ouverte. Le volume d'air disponible dans le réservoir est de l'ordre de 20 litres. Puis l'enceinte est purgée par mise en dépression à 300 mbar (30 kPa) à l'aide de la pompe 41 du circuit de mise en dépression 40. Cette opération est répétée jusqu'à atteindre la température souhaitée, permettant en particulier à un opérateur d'accéder à l'enceinte sans risque de brûlure.

A pression atmosphérique, la presse 70 est activée pour une remontée à la vitesse rapide V2 du plateau 5 jusqu'à son point haut maximum. La porte peut alors être ouverte pour décharger le bac ou charger une nouvelle quantité de déchets à traiter. En l'absence d'un opérateur, un état de veille peut être enclenché, avec rafraîchissement de l'enceinte.

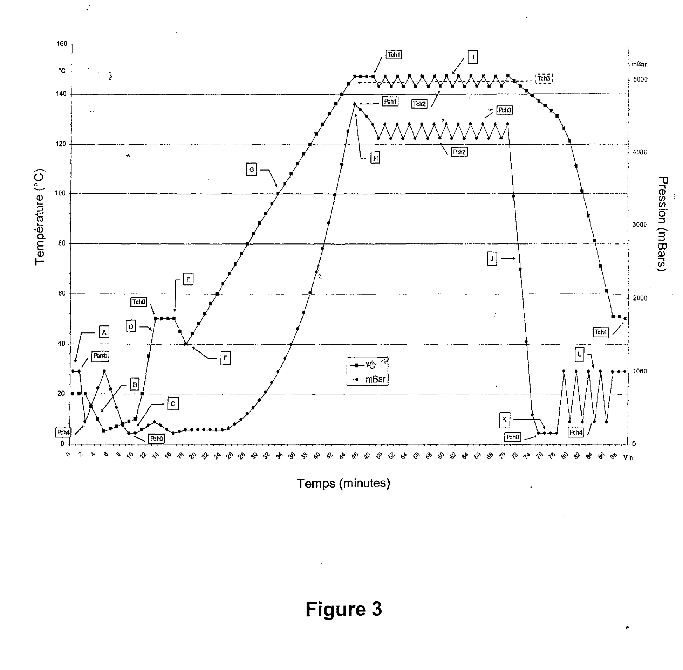

La figure 3 illustre l'évolution des conditions de température et de pression au cours d'un cycle de traitement, avec :
A : Evaluation déchets ; B : Rafraîchissement ; C Dépression ; D : Préchauffage
E : Dépression ; F : Injection d'eau ; G : Chauffage ; H : Avant-palier ; I : Palier ;
J : Déshydratation ; K : Tassement ; L :Refroidissement.

| | |
|---|---|
| Tch0 | Seuil de température de préchauffage de chambre pour élimination des vapeurs d'alcool |
| Tch1 | Seuil maxi de température chambre en avant palier et régulation de palier |
| Tch2 | Seuil mini de température de chambre en régulation de palier |
| Tch3 | Seuil moyen de température de chambre en régulation de palier |
| Tch4 | Température finale de l'enceinte en fin de cycle |
| Pamb | Pression ambiante. Mesurée lorsque la porte est ouverte |
| Pch0 | Pression minimum en cas de dépression chambre |
| Pch1 | Seuil de pression de chambre en palier = PVsat eau à Tch1 |
| Pch2 | Seuil de pression de chambre en palier = PVsat eau à Tch2 |
| Pch3 | Seuil pression de chambre en régulation de palier pour équilibre PVsat/PVsec=PVsat eau à Tch3 |
| Pch4 | Pression de chambre en dépression pour rafraîchissement/refroidissement |

### EXEMPLE 3 : CALCUL DU VOLUME D'EAU AJOUTE

Une densité de vapeur maximum est un élément important dans l'efficacité du procédé et la qualité de désinfection. Une vapeur sèche signifie que toute l'eau présente dans l'enceinte est passée de l'état liquide à l'état de vapeur. La pression de vapeur est inférieure à la pression de vapeur saturante à égale température. La densité de vapeur n'est alors pas à son maximum. Un état de vapeur saturante est obtenu lorsqu'il subsiste un équilibre entre liquide et vapeur. L'humidité relative est alors proche de 100% et c'est le compromis idéal pour une bonne qualité de désinfection.

Pour connaître la quantité minimum d'eau permettant de passer à l'état de vapeur saturante, on calcule le volume d'eau qui, à une température et une pression données se serait totalement transformée en vapeur dans le volume de l'enceinte. En assimilant la vapeur d'eau à un gaz parfait et l'eau étant supposée pure, les équations thermodynamiques ont permis de calculer que, pour une cuve de 80 litres seulement 200 cm³ d'eau pure sont nécessaires pour atteindre le seuil de vapeur sèche ou saturante (ou 440 cm³ pour une cuve de 180 litres).

Ce calcul, qui permet d'avoir une évaluation des quantités entrant en jeu, s'est avéré pouvoir être valablement utilisé comme base de fonctionnement en routine de l'appareil Le volume que doit occuper la vapeur est en fait inférieur au volume brut de l'enceinte car les déchets occupent une bonne partie de ce volume. Finalement, la quantité d'eau à ajouter dans différents cas ne s'écarte que faiblement des valeurs théoriques, et on peut adopter l'approximation selon laquelle, même si les déchets sont totalement secs, l'ajout de 200 cm³ d'eau suffira à fournir à une pression de vapeur saturante dans une enceinte de 80 litres (ou 440 cm³ dans une enceinte de 180 litres).

## Revendications

1. Appareil de pré-traitement des déchets à risque par désinfection comprenant :
- une enceinte (1) de traitement pouvant accueillir un bac (100) apte à contenir les déchets, dotée d'une porte étanche (2),
- des moyens de chauffage des déchets par rayonnement électromagnétique (3), et
- des moyens d'alimentation en eau (4) de l'enceinte,
**caractérisé en ce qu'**il comprend des moyens d'injection d'une quantité d'eau sur les déchets présents dans ledit bac et des moyens de compression desdits déchets, lesdits moyens de compression comportant un plateau mobile (5) dans lequel sont ménagés lesdits moyens d'injection.

2. Appareil selon la revendication 1, **caractérisé en ce que** lesdits moyens d'injection d'une quantité d'eau ménagés dans le plateau mobile (5) comprennent un conduit (6) et au moins une buse (7) débouchant à la surface inférieure dudit plateau mobile, ledit conduit étant relié à un circuit d'amenée d'eau (20) par un tuyau extensible (9).

3. Appareil selon l'une des revendications 1 ou 2, **caractérisé en ce que** les moyens d'alimentation en eau de l'enceinte (1) comprennent un réservoir (10) réfrigéré et un circuit d'amenée d'eau (20) muni d'une pompe d'injection (21) et d'une électrovanne (22).

4. Appareil selon l'une des revendications précédentes, **caractérisé en ce qu'**il comprend un circuit d'apport d'air froid (30) dans l'enceinte (1), muni d'une électrovanne (31).

5. Appareil selon l'une des revendications 3 ou 4, **caractérisé en ce que** le réservoir (10) est doté d'un module à effet Peltier (11), d'un capteur de température (12) et de détecteurs des niveaux minimum et maximum autorisés de l'eau (13).

6. Appareil selon la revendication précédente, **caractérisé en ce que** le circuit d'apport d'air froid (30) dans l'enceinte (1) prend son origine dans la partie supérieure du réservoir (10) réfrigéré, au-dessus du niveau de l'eau.

7. Appareil selon l'une quelconque des revendications précédentes, **caractérisé en ce qu'**il comprend des moyens de contrôle et de régulation de la pression interne de l'enceinte (1), lesdits moyens comprenant :
- au moins un capteur de la pression interne (14),
- un circuit de mise en dépression (40) de l'enceinte muni d'une pompe à vide (41) et d'une électrovanne (42),
- un circuit d'extraction (50) de la vapeur formée dans l'enceinte (1), et
- un circuit de sécurité (60) muni d'une soupape de surpression (61).

8. Appareil selon la revendication précédente, **caractérisé en ce que** le circuit d'extraction de la vapeur (50) pénètre dans le réservoir (10) de sorte que son orifice de sortie (51) débouche sous le niveau de l'eau.

9. Appareil selon l'une quelconque des revendications précédentes, **caractérisé en ce que** les moyens de compression comprennent une presse électrique (70) dont le plateau mobile (5) a une surface d'appui inférieure à l'ouverture du bac (100).

10. Appareil selon la revendication précédente, **caractérisé en ce que** le plateau mobile (5) comprend en outre des buses latérales (7') aptes à projeter de l'eau sur les parois latérales de l'enceinte (1).

11. Appareil selon l'une des revendications précédentes, **caractérisé en ce qu'**il comporte des moyens de détection de la présence (110) d'un bac (100) et de son degré de remplissage, lesdits moyens de détection étant constitués d'une plaque support (111) sur laquelle ledit bac est destiné à être posé, ladite plaque reposant sur des ressorts tarés (112) avec butée (113), et des moyens de mesure et d'analyse de la force de pression exercée par le plateau mobile (5) de la presse (70) en fonction de son déplacement.

12. Appareil selon l'une quelconque des revendications précédentes, **caractérisé en ce qu'**il comprend en outre un ou plusieurs des moyens suivants :
- un capteur de température dans l'enceinte,
- des moyens de contrôle des paramètres de fonctionnement de l'appareil,
- des moyens d'acquisition des paramètres de fonctionnement de l'appareil,
- des moyens informatiques de pilotage avec panneau de contrôle visuel.

13. Procédé de pré-traitement des déchets infectieux par désinfection, **caractérisé en ce qu'**il comprend essentiellement les étapes consistant à :
- déposer les déchets à traiter dans un bac (100) placé dans l'enceinte (1) d'un appareil de pré-traitement selon l'une des revendications précédentes, qui comprend des moyens d'injection d'une quantité d'eau sur les déchets et des moyens de compression desdits déchets comportant un plateau mobile (5) dans lequel sont ménagées lesdits moyens d'injection ;
- fermer la porte étanche (2) de l'enceinte (1) ;
- injecter une quantité d'eau sur les déchets présents dans le bac (100) par les moyens d'injection ménagés dans le plateau mobile (5) ;
- soumettre les déchets à un rayonnement électromagnétique jusqu'à réalisation des conditions de palier température - pression - temps adéquates pour réaliser la désinfection ;
- compresser les déchets ainsi traités en abaissant le plateau mobile (5) des moyens de compression dans le bac ;
- faire refroidir l'enceinte (1) avant de déverrouiller la porte.

14. Procédé selon la revendication précédente, **caractérisé en ce qu'**il comprend, avant l'étape c) d'injection de l'eau, une étape c₂) d'élimination des composés dont la tension de vapeur est supérieure à celle de l'eau, au cours de laquelle on réduit la pression dans l'enceinte (1), puis on soumet les déchets à un rayonnement électromagnétique jusqu'à vaporisation desdits composés, et on purge les vapeurs formées par une nouvelle réduction de la pression dans l'enceinte.

15. Procédé selon la revendication précédente, **caractérisé en ce que** l'étape c₂) comprend une étape préalable c₁) de rafraîchissement de l'enceinte (1), au cours de laquelle on purge d'abord l'air de l'enceinte (1) à l'aide d'un circuit de mise en dépression (40) muni d'une la pompe à vide (41), puis on apporte de l'air froid par un circuit d'apport d'air froid (30).

16. Procédé selon la revendication précédente, **caractérisé en ce que**, à l'étape c), on injecte une quantité d'eau fixe, prédéterminée en fonction des dimensions de l'appareil.

17. Procédé selon l'une des revendications 13 à 16, **caractérisé en ce que**, durant l'étape d) de désinfection, la pression de vapeur dans l'enceinte (1) est maintenue à une valeur sensiblement constante en évacuant une partie de la vapeur formée.

18. Procédé selon l'une des revendications 13 à 17, **caractérisé en ce que**, au début de l'étape d) de désinfection, la pression dans l'enceinte (1) est maintenue à la pression de vapeur saturante minimum, de sorte que toute l'eau soit transformée en vapeur.

19. Procédé selon l'une des revendications 13 à 18, **caractérisé en ce que**, l'eau injectée dans l'enceinte (1) à l'étape c) est stockée dans un réservoir (10) réfrigéré à une température inférieure à la température ambiante, de préférence inférieure à 10°C.

20. Procédé selon la revendication précédente, **caractérisé en ce qu'**à l'étape c₁) de rafraîchissement de l'enceinte (1), l'air froid est prélevé dans la partie supérieure du réservoir réfrigéré (10), au-dessus du niveau de l'eau, par l'effet de la différence de pression entre le réservoir (10) et l'enceinte (1).

21. Procédé selon l'une des revendications 13 à 20, **caractérisé en ce que** durant l'étape d) de désinfection, la vapeur d'eau produite dans l'enceinte (1) est évacuée par un circuit d'extraction (50) de la vapeur plongeant dans le réservoir (10) réfrigéré de sorte qu'elle se condense et s'y déverse.

22. Procédé selon l'une des revendications 13 à 21, **caractérisé en ce qu'**il comprend, immédiatement après l'étape d) de désinfection, une étape d₁) de déshydratation des déchets sous pression réduite.

23. Procédé selon l'une des revendications 13 à 22, **caractérisé en ce que** l'étape e) de compression est réalisée par descente du plateau mobile (5) de la presse (70) à l'intérieur du bac (100).

24. Procédé selon l'une des revendications 13 à 23, **caractérisé en ce que** durant l'étape f) de refroidissement des déchets traités, on purge l'air de l'enceinte (1) à l'aide du circuit de mise en dépression (40), puis on apporte de l'air froid prélevé dans la partie supérieure du réservoir (10) réfrigéré, ces opérations étant répétées jusqu'à atteindre la température finale souhaitée.

25. Procédé selon l'une des revendications 13 à 24, **caractérisé en ce qu'**il comprend une étape de détection de la présence d'un bac (100) et de son degré de remplissage, à l'aide d'une plaque support (111) sur laquelle ledit bac est supposé être posé, celle-ci reposant sur des ressorts tarés (112) avec butée (113), et des moyens de mesure et d'analyse de la force de pression exercée par le plateau (5) de la presse mobile (70) lors de son déplacement.

26. Procédé selon l'une des revendications 13 à 25, **caractérisé en ce qu'**il comprend entre deux cycles de traitement, une étape de nettoyage des parois latérales de l'enceinte
(1) par descente du plateau mobile (5) et projection d'eau par des buses latérales (7').
